# EUROPÄISCHE PATENTSCHRIFT

(11) **EP 0 635 476 B1**
(45) Veröffentlichungstag und Bekanntmachung des Hinweises auf die Patenterteilung: **22.10.1997**
(21) Anmeldenummer: 94110540.5
(22) Anmeldetag: 06.07.1994
(51) Int. Cl.: C07C 68/02, C07C 69/96

(54) **Verfahren zur Herstellung von Arylcarbonaten**
Process for the preparation of aryle carbonates
Procédé de préparation carbonates d'aryle

(30) Priorität: 19.07.1993 DE 4324153
(43) Veröffentlichungstag der Anmeldung: 25.01.1995
(73) Patentinhaber: BAYER AG, 51368 Leverkusen (DE)
(72) Erfinder: Ooms, Pieter, Dr., D-47800 Krefeld (DE); Schön, Norbert, Dr., D-47800 Krefeld (DE); Buysch, Hans-Josef, Dr., D-47809 Krefeld (DE)

(56) Entgegenhaltungen:
- US-A- 2 362 865
- US-A- 5 167 946
- US-A- 5 239 105

## Beschreibung

Die vorliegende Erfindung betrifft ein Verfahren zur Herstellung von Carbonaten mit aromatischen Estergruppen durch Umsetzung von aromatischen Monohydroxyverbindungen mit Phosgen oder Chlorkohlensäureestern aromatischer Monohydroxyverbindungen unter Abspaltung von Chlorwasserstoff in Gegenwart von Alumosilikaten als heterogene Katalysatoren.

Es ist bekannt, daß die Arylcarbonate durch Phasengrenzflächenphosgenierung (Schotten-Baumann-Reaktion) von aromatischen Hydroxyverbindungen gewonnen werden können. Dabei wirkt sich die Verwendung von Lösungsmitteln und Natronlauge nachteilig aus, da durch die Lauge eine teilweise Verseifung von Phosgen oder Chlorkohlensäureester stattfinden kann. In jedem Falle bringen die großen Mengen von anfallendem Natriumchlorid eine Abwasserbelastung mit sich. Ferner muß Sorge für die Lösungsmittel-Rückgewinnung getragen werden, wobei ein effektiver Umweltschutz gesichert sein muß.

Man hat daher eine Kondensation ohne Mitverwendung von Lösungsmitteln in Gegenwart von Tetramethylammoniumhalogeniden als homogene Katalysatoren vorgeschlagen (US-PS 2.837.555). Dabei sind aber die benötigten Katalysatormengen relativ groß. Man muß in der Regel mit 5 - 7 Gewichtsprozent Katalysator, bezogen auf die eingesetzte Phenolmenge, arbeiten, um wirtschaftliche Reaktionsgeschwindigkeiten zu erhalten; die Reaktionstemperaturen von 180°C - 215°C bringen die Gefahr einer Zersetzung der thermolabilen Tetramethylammoniumhalogenide mit sich. Der Katalysator muß ferner anschließend durch Waschen mit Wasser entfernt werden, wodurch seine Rückgewinnung erheblich erschwert wird. Darüber hinaus wird weit über die stöchiometrisch notwendige Menge an Phosgen verbraucht.

Nach einem weiteren Verfahren (US-PS 3.234.263) werden Diphenylcarbonate durch Erhitzen von Phenylchlorkohlensäureestern in Gegenwart großer Mengen (Erd)Alkaliverbindungen oder tertiären Stickstoffbasen als Katalysatoren erhalten. Dieses Verfahren hat jedoch den Nachteil, daß man hohe Temperaturen anwenden muß und die Katalysatoren wie (Erd)Alkaliverbindungen teilweise gelost werden, um auch nur annähernd auf wirtschaftlich vertretbare Reaktionszeiten zu kommen. Bei diesem Verfahren geht die Hälfte des ursprünglich eingesetzten Phosgens in Form von CO₂ verloren. Außerdem muß man in einem vorgelagerten separaten Verfahrensschritt die Chlorkohlensäureester synthetisieren.

Nach der CA-A-2 058 359 (US-A-5 167 946) erhält man Diarylcarbonate durch Phosgenierung von aromatischen Hydroxyverbindungen in Gegenwart von Aluminiumverbindungen, die unter den Reaktionsbedingungen zumindest teilweise löslich sind, bzw. in lösliche Aluminiumhalogenide übergehen und offensichtlich auf diese Art als homogene Katalysatoren wirken (vgl. US-A-2 362 865, Sp. 1, Z. 45-53). Besonders bevorzugt ist darum auch Aluminiumtrichlorid (Löslichkeit). Obwohl sehr gute Ausbeuten erhalten werden, ist es schwierig die Katalysatoren von den Produkten zu trennen. Man muß sogar bei Destillationen mit einer gewissen Flüchtigkeit dieser Verbindungen und auch mit thermischen Zersetzungen durch diese Aluminiumverbindungen rechnen, die zu Verunreinigungen, Qualitätsminderungen und Ausbeuteeinbußen führen. Ähnliches gilt für das Verfahren der US-A-2 362 865, die noch die Verwendung von metallischen Titan, Eisen, Zink und Zinn oder in Form ihrer löslichen Salze, besonders der Chloride und Phenolate als Katalysatoren nennt.

Somit erscheint es sinnvoll, heterogene, nicht lösliche Katalysatoren zu verwenden, die eine Aufarbeitung des Reaktiongsgemisches wesentlich erleichtern. Auch dazu wurden Vorschläge gemacht. So wird nach der Lehre der EP-A 516 355 vor allem Aluminiumtrifluorid empfohlen, das gegebenenfalls auf Träger wie Alumosilikate aufgebracht wird. Die Synthese von Aluminiumfluorid ist jedoch mit der Handhabung von Fluor oder Flußsäure, sehr toxischen Verbindungen, damit auch mit aufwendigen und teuren Apparataten verbunden.

Die Aufgabe der Erfindung bestand also darin, einfacher zugängliche, wirksame heterogene Katalysatoren zu entwickeln.

Es wurde nun gefunden, daß Alumosilikate ausgezeichnete Katalysatoren für die Umsetzung von Phosgen oder Chlorkohlensäureestern mit aromatischen Hydroxyverbindungen darstellen. Dies ist besonders überraschend und unerwartet, weil soche Verbindungen nach der Lehre der EP-A 516 355 als inert bekannt sind. Eine katalytische Aktivität im Sinne der vorliegenden Erfindung wird nicht berichtet.

Der Gegenstand der vorliegenden Erfindung ist demnach ein Verfahren zur Herstellung von Arylcarbonaten durch Umsetzung von aromatischen Monohydroxyverbindungen mit Phosgen oder Chlorkohlensäureestern aromatischer Monohydroxyverbindungen, das dadurch gekennzeichnet ist, daß man bei einer Temperatur im Bereich von 50-350°C, bei einem Druck von 0,2 bis 20 bar in Gegenwart von Alumosilikaten als heterogenem Katalysator arbeitet.

Das erfindungsgemäße Verfahren hat den großen Vorteil, daß man den Katalysator sehr leicht abtrennen kann und keine Verunreinigungen im rohen Reaktionsprodukt verbleiben. Die Aufarbeitung wird dadurch wesentlich vereinfacht.

Aromatische Monohydroxyverbindungen für das erfindungsgemäße Verfahren sind solche der Formel

Ar¹-OH (I),

worin
- Ar¹: Phenyl, Naphtyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen oder heterocyclischen Reste durch 1 oder 2 Substituenten wie geradkettiges oder verzweigtes C₁-C₄-Alkyl, geradkettiges oder verzweigtes C₁-C₄-Alkoxy, die mit Phenyl, Cyano und/oder Halogen (z.B. F, Cl, Br) substituiert sein können und wobei weiterhin die heterocyclischen Reste mit einem ankondensierten Benzolkern verbunden sein können.

Beispiele für aromatische Monohydroxyverbindungen der Formel (I) sind:
Phenol, o-, m- und p-Kresol, o-, m- und p-Isopropylphenol, die entsprechenden Halogen- bzw. Alkoxyphenole, wie p-Chlorphenol bzw. p-Methoxyphenol, ferner Monohydroxyverbindungen des Naphtalins, Anthracens und Phenanthrens, weiterhin 4-Hydroxy-pyridin und Hydroxychinoline. Vorzugsweise werden gegebenenfalls substituierte Phenole, ganz besonders bevorzugt Phenol selbst, eingesetzt.

Das erfindungsgemäße Verfahren kann sowohl mit Phosgen als auch mit Chlorkohlensäureestern aromatischer Monohydroxyverbindungen durchgeführt werden Für den Fall der Durchführung mit Phosgen entsteht zunächst der Chlorkohlensäureester, der mit weiterer im Reaktionsgemisch vorhandener aromatischer Monohydroxyverbindung zum Diarylcarbonat umgesetzt wird.

Geht man von Chlorkohlensäureestern und einer aromatischen Monohydroxyverbindung aus, können symmetrische oder unsymmetrische Carbonate erhalten werden.

Geeignete aromatische Chlorkohlensäurester für das erfindungsgemäße Verfahren sind demnach solche der Formel (II)

Ar-OCOCl (II),

worin Ar die bei Formel (I) angegebene Bedeutung hat.

Geeignete Alumosilikate als heterogene Katalysatoren sind Zeolithe, Schichtsilikate und synthetische Alumosilikate, die weder Zeolith- noch Schichtsilikat-Strukturen besitzen.

Bei den Zeolithen handelt es sich um kristalline, synthetische oder natürliche vorkommende Alumosilikate, mit Gerüststruktur (siehe D.W. Breck in "Zeolithe Molecular Sievers", Wiley Interscience, 1974, S. 133-180; Ullmanns Enzyklopädie der technischen Chemie, 4. Auflage, Band 17, S. 9-18, Verlag Chemie, Weinheim, New York).

Die Zeolithe können durch die allgemeine Formel (III)

M_{2/n}O · Al₂O₃ · xSiO₂ x yH₂O (III),

in welcher
M für Kationen wie Protonen oder beliebige Metallkationen des Periodensystems der Elemente nach Mendelejew steht und
n für die Wertigkeit des Kations steht,
x für das Mol-Verhältnis SiO₂/Al₂O₃ steht, wobei x eine Zahl von 1,0 - 50,0, bevorzugt 2,0 - 25,0 sein kann und
y für eine Zahl von 0-9 steht.

Als Metallkationen seien beispielsweise genannt: Na, K, Ca, Mg, Lanthaniden, Ti, Sn, Zn, Fe, Ni, Co, Cu, Nb, Ta, Zr usw.

Geeignet für das erfindungsgemäße Verfahren sind Zeolithe der Struktur A, X, Y (Faujasit-Typ), L, Zeolithe vom Pentasiltyp wie ZSM 5, 11, 22, 23, Mordenit, Offretit, Phillipsit, Sodalith, Omega und zeolithähnliche Materialien wie AlPO's und SAPO's, besonders geeignet sind Zeolithe der Struktur A, des Faujasittyps wie X, Y ferner L, ZSM 5, ZSM II, Mordenit, Offretit, Omega, SAPO 5, 11, 34 und AlPO 5, 11, ganz besonders geeignet sind Zeolithe der Struktur A, X, Y, ZSM 5, Mordenit, SAPO 5, 11, 34 und AlPO 5 und 11. Sie können einzeln oder als Gemisch eingesetzt werden.

Die erfindungsgemäß einzusetzenden Schichtsilikate sind bekannt, z.B. aus Kirk-Othmer "Encyclopedia of Chemical Technology" 2nd Ed. 1964, Vol. 5, s. 541-561. In diesem Artikel werden sie klassifiziert:
A) Kaolin-Typ wie Kaolinit, Dickerit, Nacrit (alle Al₂O₃ x SiO₂ x 2 H₂O) oder Anauxit (Al₂O₃ x 3 SiO₂ x 2 H₂O) oder Halloysit (Al₂O₃ x 2 SiO₂ x 2 H₂O) oder Endellit (Al₂O₃ x 2 SiO₂ x 4 H₂O) sowie
B) die durch Erhitzen aus den Kaolintypen hergestellten Spinell-Typen,
C) Serpentin-Typen, in denen 3 Mg-Ionen 2 Al-Ionen - ausgehend von den Kaolin-Typen - ersetzt haben wie (Mg₃Si₂O₅(OH)₄) und Amesit Mg₄Al₂Si₂Al₂O₁₀(OH)₈ und eisenhaltige wie Cronstedit (Fe₂²⁺Fe³⁺) (SiFe³⁺)O₅(OH₄) sowie Chamosit (Fe²⁺, Mg)_{2.3} (Fe³⁺Al)_{0,7} (Si_{1,14}Al_{0,86})O₅(OH)₄ sowie die z.T. auch synthetisch zugänglichen Nickel- oder Kobaltspezies,
D) Alumosilikate des Montmorillonit-Typs wie z.B.
   - Montmorillonit: [Al_{1,67}Mg_{0,33}(Na_{0,33})]Si₄O₁₀(OH)₂
   - Beidellit: Al_{2,17}[Al_{0,33}(Na_{0,33})Si_{3,17}]O₁₀(OH)₂
   - Nontronit: Fe³⁺[Al_{0,33}(Na_{0,33})Si_{3,67}]O₁₀(OH)₂
   - Hectorit: Mg_{2,67}Li_{0,33}(Na_{0,33})Si₄O₁₀(OH,F)₂
   - Saponit: Mg_{1,48}Mg_{0,14}Al_{0,74}Fe³⁺][Al_{0,99}Si_{3.01}] O₁₀(OH)₂X_{0,33}
   sowie Cu²⁺, Co²⁺ oder Ni²⁺-haltige Typen (X = Halogen) wie Volkonskoit, Medmontit oder Pimelit.

Derartige Schichtsilikate können einzeln oder als Gemisch verwendet werden. Es können natürliche und synthetische Schichtsilikate eingesetzt werden.

Bevorzugt sind die als Montmorillonit-Typen" beschriebenen Schichtsilikate und besonders bevorzugt Montmorillonit selbst.

Die Alumosilikate können in ihrer ursprünglichen Form wasserhaltig oder (teil)getrocknet eingesetzt werden. Sie können auch sauer aktiviert eingesetzt werden. Die saure Aktivierung wird z.B. durch Behandlung mit Säuren, bevorzugt Mineralsäuren, vorgenommen.

Es können auch beliebige Mischungen der vorgenannten Zeolithe und/oder Schichtsilikate eingesetzt werden.

Synthetische Alumosilikate, die weder Zeolithe noch Schichtsilikate darstellen sind beispielsweise "pillared clays", wie in Mat. Res. Soc. Synp. Proc., Bd. 111, S. 257 ff. 1988, in Applied Clay Science, Bd. 2, S. 309 ff 1987 oder NATO ASI Ser., ser. C. Bd. 231, S. 271 ff. 1988 beschrieben oder Produkte, die durch gemeinsame Fällung von hydrolysierbaren Aluminium- u. Siliciumverbindungen im wäßrigen Medium, gegebenenfalls in Gegenwart von inerten Trägern oder Dispergierhilfsmitteln und anschließende Calcinierung entstehen.

Die Katalysatoren können als Pulver oder Formkörper eingesetzt und nach der Umsetzung durch Filtration, Sedimentation oder Zentrifugieren abgetrennt werden. Für den Fall der Anordnung als Festbett werden die Alumosilikate vorzugsweise als Formkörper wie Kugeln, Zylinder, Stäbchen, Hohlzylinder, Ringe usw. eingesetzt.

Die genannten Alumosilikat-Katalysatoren werden beim Arbeiten mit suspendiertem Katalysator in Rührgefäßen oder Blasensäulen in Mengen von 0,5 bis 100 Gew.-%, bevorzugt von 5 bis 100 Gew.-% und besonders bevorzugt von 5 bis 50 Gew.-%, bezogen auf die eingesetzte Menge an Monohydroxyverbindung, verwendet.

Im Falle einer kontinuierlichen Fahrweise im Gegen- oder Gleichstrom oder in der Rieselphase am Festbettkatalysator werden Katalysatorbelastungen von 0,1 bis 20 g aromatische Hydroxyverbindung pro g Katalysator pro Stunde, bevorzugt von 0,2 bis 10 g · g⁻¹·h⁻¹ und besonders bevorzugt von 0,2 bis 5 g · g⁻¹·h⁻¹ verwendet.

Die in diskontinuierlichen Versuchen verwendeten Alumosilikate können bei gleichen Einsatzstoffen ohne Reinigung wiederholt eingesetzt werdend Bei einem Wechsel der Einsatzstoffe werden die Alumosilikate zweckmäßig durch Extrahieren mit inerten Lösungsmitteln, wie sie beispielsweise weiter unten als Reaktionsmedien genannt sind, oder mit Alkoholen, wie Methanol, Ethanol, Isopropanol oder Butanol, mit Estern oder Amiden der Essigsäure oder durch Behandlung mit überhitztem Wasserdampf oder Luft gereinigt.

Bei kontinuierlicher Arbeitsweise können die eingesetzte Alumosilikate über lange Zeit im Reaktor verbleiben.

Eine Regenerierung lohnt sich in der Regel nicht; sie kann jedoch durch Überleiten von überhitztem Wasserdampf gegebenenfalls unter Zugabe von untergeordneten Mengen an Luft (etwa 0,1 bis 20 Gew.-%, bezogen auf die eingesetzte Wasserdampfmenge) bei 150 bis 800°C oder durch Überleiten von 0,01 bis 5 Gew.-% Sauerstoff enthaltenden Verdünnungsgasen wie Stickstoff, Kohlenmonoxid oder Kohlendioxid oder durch Kohlendioxid allein bei 200 bis 800°C erfolgen. Die bevorzugte Regenerierungstemperatur liegt bei 250-700°C, besonders bevorzugt bei 250 bis 600°C.

Das erfindungsgemäße Verfahren wird bei einer Temperatur im Bereich von 50-350°C, bevorzugt 100-300°C, besonders bevorzugt 100-250°C durchgeführt. Während der Durchführung des erfindungsgemäßen Verfahrens kann die Temperatur im genannten Bereich verändert, in bevorzugter Weise erhöht werden.

Das erfindungsgemäße Verfahren wird bei einem Druck von 0,2-20 bar, bevorzugt 1,0-5 bar durchgeführt.

Man kann das erfindungsgemäße Verfahren unter Mitwirkung von Lösungsmitteln wie aliphatischen und aromatischen Kohlenwasserstoffen wie Pentan, Hexan, Octan, Benzol, Xylolen, Diethylbenzol, Alkylnaphthalinen, Biphenyl, halogenierten Kohlenwasserstoffen wie Dichlormethan, Trichlorethylen usw. durchführen.

Vorzugsweise wird das Verfahren in der Schmelze durchgeführt, beispielsweise, indem man in eine Suspension von Alumosilikat in einer Schmelze der aromatischen Monohydroxyverbindung der Formel (I) Phosgen oder einen Chlorkohlensäureester der Formel (II) einleitet und nach Beendigung der Reaktion den Katalysator durch z.B. Filtration, Zentrifugiere abtrennt.

Eine weitere bevorzugte Ausführungsform der Synthese ist die Begasung einer Schmelze der aromatischen Monohydroxyverbindung der Formel (I) mit darin suspendiertem Alumosilikatkontakt mit Phosgen oder Phosgen-Chlorwasserstoff-Gemischen oder mit Chlorkohlensäureestern der Formel (II) in einer kontinuierlich arbeitenden Blasensäule bzw. Blasensäulen- Kaskade.

Eine weitere bevorzugte Durchführungsform ist das Gleichstromverfahren, bei dem aromatische Hydroxyverbindungen der Formel (I) und Phosgen bzw. Chlorkohlensäureester der Formel (II) im Gleichstrom beispielsweise von oben her auf eine in einem Rohr angeordnete Katalysatorschüttung aufgebracht und unten am Fuß des Rohres Chlorwasserstoff und Phosgenierungsprodukte abgezogen werden.

Eine weitere bevorzugte Ausführungsform mit besonders günstigen Ergebnissen ist die Durchführung der erfindungsgemäßen Umsetzung in der Rieselphase, wobei die aromatische Monohydroxyverbindung der Formel (I) als Schmelze oder in Form einer Lösung oben auf ein Bett von Alumosilikaten gegeben wird und diesem Flüssigkeitsstrom von unten ein Strom von Phosgen oder Chlorkohlensäureester der Formel (II) entgegengeschickt wird Zweckmäßig wird diese Ausführungsform in einem senkreicht stehenden Rohrreaktor durchgeführt, die auch Zwischenböden zur verbesserten Verteilung von Gas-und Flüssigkeitsstrom enthalten kann.

Das molare Verhältnis der Reaktionspartner beträgt aromatische Monohydroxyverbindung der Formel (I) zu Phosgen wie 0,5-3:1, bevorzugt 1,5-3:1. Das äquivalente molare Verhältnis ist in diesem Fall 2:1.

In entsprechender Weise wird die aromatische Monohydroxyverbindung der Formel (I) mit einem Chlorkohlensäureester der Formel (II) im Molverhältnis von 0,25 bis 4:1, bevorzugt 0,8-1,5:1 umgesetzt. In diesem Fall ist das molare Verhältnis 1:1.

Das durch heterogene Katalyse gewonnene rohe aromatische Carbonat ist häufig schon sehr rein und kann nach Entgasung von restlichem Chlorwasserstoff oder anderen flüchtigen Stoffen bereits in dieser Form für viele Zwecke verwendet werden. Für anspruchsvollere Verwendung kann das Carbonat gegebenenfalls z.B. durch Destillation oder Kristallisation weiter gereinigt werden.

Die Arylcarbonate sind z.B. geeignete Vorprodukte zur Herstellung von Phenylurethanen, Polycarbonaten und Wirkstoffen.

### Beispiele

### Beispiel 1

In einem Planschlifftopf mit Strombrechern, Begasungsrührer und Ruckflußkühler wurden 141 g (1,50 mol) Phenol in Gegenwart von 14,1 g (10 Gew.-% bez. auf Phenol) pulverförmigem H-SAPO 5 mit 0,75 mol/h Phosgen begast. Nach etwa 2 h Reaktion bei 140°C betrug der Phenolumsatz 7,1 %, wobei 4,9 g Chlorameisensäurephenylester und 8,0 g Diphenylcarbonat gebildet wurden. Die Selektivität zu den Carbonaten war größer als 99 %.

### Beispiel 2

Das Beispiel 1 wurde bei 160°C wiederholt. Nach 2 h Reaktionszeit betrug der Phenolumsatz 11,9 %, wobei 0,6 g Chlorameisensäurephenylester und 18,6 g Diphenylcarbonat gebildet wurden. Die Selektivität zu den Carbonaten war größer als 99 %.

### Beispiel 3

Das Beispiel 1 wurde mit 14,1 g pulverförmigem H-ZSM 5 bei 160°C wiederholt. Nach 2 h Reaktionszeit betrug der Phenolumsatz 8,3 %, wobei 0,5 g Chlorameisensäurephenylester und 12,4 g Diphenylcarbonat gebildet wurden. Die Selektivität zu den Carbonaten war ca. 96 %.

### Beispiel 4

Das Beispiel 1 wurde mit 14,1 g pulverförmigem H-Y bei 160°C wiederholt. Nach 2 h Reaktionszeit betrug der Phenolumsatz 11,0 %, wobei < 0,1 g Chlorameisensäurephenylester und 17,5 g Diphenylcarbonat gebildet wurden. Die Selektivität zu den Carbonaten war ca. 99 %.

### Beispiel 5 (zum Vergleich)

Das Beispiel 1 wurde ohne Zusatz von Alumosilikat wiederholte Nach 4 h bei 160°C betrug der Phenolumsatz < 0,2 %.

### Beispiel 6

In einem Dreihalskolben mit Thermometer und Rückflußkühler erhitzt man ein Gemisch aus 9,4 g (0,1 Mol) Phenol und 15,7 g (0,1 Mol) Chlorameisensäurephenylester in Gegenwart von 0,94 g (10 Gew.-% bezogen auf Phenol) pulverförmigem H-ZSM 5. Nach 2 h bei 160°C wird ein Phenolumatz von 72 % zu Diphenylcarbonat gefunden. Die Selektivität war > 99 %.

### Beispiel 7

Beispiel 6 wurde wiederholt, jedoch mit 0,94 g pulverförmigen H-Y und 3 h bei 120° C; Phenolumsatz zu Diphenylcarbonat 55 %, Selektivität > 99 %.

### Beispiel 8

Beispiel 6 wurde wiederholt, jedoch mit 0,94 g pulverförmigem H-Y 1 h 160° C; Phenolumsatz zu Diphenylcarbonat 70 %; Selektivität > 99 %.

### Beispiel 9

Beispiel 6 wurde wiederholt, jedoch mit 0,94 g pulverförmigem SAPO 11 und 2 h 160° C; Phenolumsatz zu Diphenylcarbonat 64 %, Selektivität > 99 %.

### Beispiel 10

Beispiel 6 wurde wiederholt, jedoch mit 0,94 g pulverförmigem ALPO 11 und 3 h 160° C; Phenolumsatz zu Diphenylcarbonat 91 %, nach 5 h 97 %; Selektivität > 99 %.

### Beispiel 11

Beispiel 6 wurde wiederholt, jedoch mit 0,94 g pulverförmigem Montmorillonit KSF/O (Südchemie) und 5 h bei 160° C; Phenolumsatz 55 %, Selektivität > 99 %.

### Beispiel 12

Beispiel 6 wurde wiederholt, jedoch mit 0,94 g pulverförmigem Na-X und 3 h bei 160° C; Phenolumsatz 90 %, Selektivität > 99 %.

### Beispiel 13

Beispiel 6 wurde wiederholt, jedoch mit 0,94 g pulverförmigem Na-A und 3 h bei 160° C; Phenolumsatz 56 %, Selektivität > 99 %.

## Patentansprüche

1. Verfahren zur Herstellung von Arylcarbonaten durch Umsetzung von aromatischen Monohydroxyverbindungen mit Phosgen oder Chlorkohlensäureestern aromatischer Monohydroxyverbindungen, dadurch gekennzeichnet, daß man die Umsetzung bei einer Temperatur im Bereich von 50-350°C unter einem Druck von 0,2 bis 20 bar in Gegenwart von einem oder mehreren Alumosilikaten als heterogene Katalysatoren durchführt.

2. Verfahren nach Anspruch 1, dadurch gekennzeichnet, daß ein oder mehrere Alumosilikate ausgewählt aus der Gruppe der Zeolithe der Formel
M_{2/n}O · Al₂O₃ · xSiO₂ x yH₂O
in welcher
M für Kationen wie Protonen oder Metallkationen des Periodensystems der Elemente nach Mendelejew steht,
n für die Wertigkeit des Kations steht,
x für das Mol-Verhältnis SiO₂/Al₂O₃ steht, wobei
x eine Zahl von 1,0-50,0 sein kann, und
y für eine Zahl von 0 - 9 steht,
oder zeolithähnliche Verbindungen wie ALPO's und SAPO's oder Schichtsilikate des Kaolin-, Serpentin-, Montmorillonit- und Bentonit-Typs oder "pillared clays" oder SiO₂/Al₂O₃-Fällungskatalysatoren in einer Menge von 0,5-100 Gew.-%, bezogen auf die Menge der Monohydroxyverbindungen, bei suspendiertem Katalysator bzw. mit Belastungen von 0,1-20 g pro g Katalysator pro Stunde bei der Anordnung als Festbettkatalysator eingesetzt wird.

## Claims

1. A process for preparing aryl carbonates by reacting aromatic monohydroxy compounds with phosgene or chloroformates of aromatic monohydroxy compounds, characterised in that the reaction is performed at a temperature in the range 50-350°C under a pressure of 0.2 to 20 bar in the presence of one or more aluminosilicates as heterogeneous catalysts.

2. A process according to Claim 1, characterised in that one or more aluminosilicates selected from the group of zeolites of the formula
M_{2/n}O · Al₂O₃ · xSiO₂ · yH₂O
in which
M represents cations such as protons or cations of metals from Mendeleev's periodic table of the elements,
n represents the valency of the cation,
x represents the molar ratio SiO₂/Al₂O₃, wherein
x may be a number from 1.0-50.0, and
y is a number from 0 - 9,
or zeolite-like compounds such as ALPOs and SAPOs or sheet silicates of the kaolin, serpentine, montmorillonite and bentonite type or "pillared clays" or SiO₂/Al₂O₃ precipitation catalysts are used in an amount of 0.5-100 wt.%, with reference to the amount of monohydroxy compounds, when using a suspended catalyst or with loads of 0.1-20 g per g of catalyst per hour when using a fixed bed arrangement for the catalyst.

## Revendications

1. Procédé de préparation de carbonates d'aryle par réaction de composés monohydroxylés aromatiques avec du phosgène ou des chlorocarbonates de composés monohydroxylés aromatiques, caractérisé en ce que l'on effectue la réaction à une température comprise entre 50 et 350°C, sous une pression de 0,2 à 20 bars, en présence d'un ou plusieurs aluminosilicates comme catalyseurs hétérogènes.

2. Procédé selon la revendication 1, caractérisé en ce que l'on utilise un ou plusieurs aluminosilicates choisis dans le groupe des zéolites de formule
M_{2/n}O · Al₂O₃ · xSiO₂ · yH₂O
dans laquelle
M représente un cation comme un proton ou un cation métallique quelconque du système périodique des éléments selon Mendeleiev,
n est égal à la valence du cation,
x représente le rapport molaire SiO₂/Al₂O₃, x pouvant être un nombre compris entre 1,0 et 50,0, et
y est un nombre de 0 à 9,
ou des composés analogues aux zéolites comme les AlPO et les SAPO ou des phyllosilicates de type kaolin, serpentine, montmorillonite et bentonite ou des "*pillared clays*", ou des catalyseurs obtenus par précipitation de SiO₂/Al₂O₃, en une quantité de 0,5 à 100 % en masse par rapport à la quantité de composé monohydroxylé dans le cas d'un catalyseur en suspension, ou avec des charges de 0,1 à 20 g par g de catalyseur et par heure lorsque le catalyseur est disposé sous forme de catalyseur en lit fixe.
